# EUROPEAN PATENT SPECIFICATION

(11) **EP 3 661 508 B1**
(45) Date of publication and mention of the grant of the patent: **22.05.2024**
(21) Application number: 17764504.1
(22) Date of filing: 02.08.2017
(51) Int. Cl.: A61K 31/4375, A61K 9/20

(54) **MANUFACTURING METHOD OF ORAL DOSAGE FORM CONTAINING BERBERINE, ORAL DOSAGE FORM CONTAINING BERBERINE AND USE THEREOF**
VERFAHREN ZUR HERSTELLUNG VON BERBERINHALTIGER ORALDOSIERUNGSFORM, BERBERINHALTIGE DOSIERUNGSFORM UND DESSEN VERWENDUNG
PROCÉDÉ DE FABRICATION D'UNE FORME POSOLOGIQUE ORALE CONTENANT DE LA BERBÉRINE, FORME POSOLOGIQUE ORALE CONTENANT DE LA BERBERINE ET UTILISATION DE CELLE-CI

(43) Date of publication of application: 10.06.2020
(73) Proprietor: Pro.Med.CS Praha A.S., 14000 Praha 4 - Michle (CZ)
(72) Inventor: BOUSKOVA, Aneta, 10000 Praha 10 (CZ); LISAL, Jiri, 182 00 Praha 8 (CZ); REZAC, Jaroslav, 277 11 Neratovice (CZ); SVOBODA, Zbynek, 503 03 Holohlavy (posta Smirice) (CZ)
(74) Representative: Hartvichova, Katerina
(86) International application number: PCT/CZ2017/050033
(87) International publication number: WO 2019/024949

(56) References cited:
- EP-A1- 3 064 200
- WO-A1-2013/063271
- WO-A1-2015/097642
- CN-A- 104 997 735
- JP-A- 2013 256 480
- US-A1- 2016 058 809
- M. LI ET AL: "Comparison of Particle Size Distributions Measured Using Different Techniques", PARTICULATE SCIENCE AND TECHNOLOGY, vol. 23, no. 3, 24 February 2007 (2007-02-24), pages 265-284, XP055269915, US ISSN: 0272-6351, DOI: 10.1080/02726350590955912
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368

## Description

### Field of Art

The present invention relates to a manufacturing method of oral dosage form containing berberine, enabling easy manipulation and processing, such as dry granulation, homogenization, compression and capsule filling of mixtures containing berberine. The present invention also relates to a composition of a mixture containing berberine, suitable for dry granulation, homogenization, tabletting and capsule filling, as well as to the use thereof.

### Background Art

Berberine is a plant isoquinoline alkaloid chemically described as 5,6-dihydro-9,10-dimethoxybenzo[g]-1,3-benzodioxolo[5,6-a]quinolizinium. This compound has a long history of medicinal use in both Chinese and Ayurvedic medicine. Berberine is present in many plants such as Hydrastis canadensis (goldenseal), Coptis chinensis (coptis or goldenthread), Phellodendron, Berberis aquifolium (Oregon grape), Berberis vulgaris (barberry), and Berberis aristata (tree tumeric).

Berberine has broad antibacterial activities. Berberine has been proven to have also other pharmacological effects including blood glucose-lowering, high cholesterol control, hypertension treatment, weight control, heart disease treatment, anti-aging, immune challenges and antiinflammatory properties. Moreover, there is increasing research on the regulation of cancer cell metabolism by berberine hydrochloride. Berberine has been shown to be effective against bacteria, protozoa and fungi.

At present, there are many oral food supplements containing berberine on the market world-wide mostly in form of capsule. As the compound has a short half-life, three times a day is generally recommended to take in oral dosage forms. Commonly recommended dose to take is 500 mg three times a day for a total of 1,500 mg per day.

Different types of oral dosage forms were described in the prior art documents. Generally and most preferably berberine is formulated in solid dosage forms. Oral liquid formulations comprising berberine are described in EP2937077 A1 patent application and EP 0227108 A2.

Some known methods of preparing berberine-containing dosage forms are summarized below.

EP 3064200 A1 discloses an oral dosage form composition comprising berberine, croscarmellose sodium, colloidal silica, anhydrous biphasic calcium phosphate, magnesium stearate and microcrystalline cellulose. The composition is used as medicament or food supplement for preventing or treating hypercholesterolaemia or hypertriglyceridaemia.

US 2016/058809 A1 and WO 2013/063271 A1 mentioned above both disclose a method of preparing a tablet comprising berberine. The method steps comprise adding artemisine, berberine and the non-active ingredients to a V-blender, starting with microcrystalline cellulose, followed by silicon dioxide, stearic acid, calcium carbonate, magnesium stearate and croscarmellose. Blending is continued for additional 20 to 30 minutes before filtering and forming the tablets.

WO 2015/097642 A1 discloses a process of mixing berberine and polyethylene glycol in solution of water and ethanol to form creamy precipitations, which are then collected, passed through a sieve, and dried in a hot air oven to form granules. The granules thus formed are then filled in capsules.

JP 2013 256480 A discloses a method of manufacturing a tablet containing the berberine tannate particles by granulation, and CN 104 997 735 A discloses a method of preparing a taste-masked pellet of berberine hydrochloride and auxiliary material through extrusion centrifugation pelleting, centrifugation lamination, or sugar coat pot rolling method.

Berberine has some important physical properties which should be taken into account when formulating the dosage forms. The compound extreme bitterness should be masked by inactive ingredients and manufacturing process, e.g. by coating. Other undesirable property is high water content, approximately 14 % (w/w) which makes the manufacturing of dry, physically stable pills difficult, as described in WO 2013063271 A1 patent application.

Generally water content of both water in the active substance itself and water as a binder added during dosage form manufacturing make formulation very challenging because mixture of berberine and water is strongly sticking on any surfaces of manufacturing equipment. Other undesirable property is very poor flow of the active substance which is connected to very fine particles needed for demanded pharmaceutical effect. Therefore usage of conventional manufacturing method such as direct compression, wet granulation but also dry granulation is very difficult. All the above mentioned disadvantages make the manufacturing process very expensive due to big losses of the material and in some cases technologically even impossible.

### Disclosure of the Invention

The present invention relates to a manufacturing method of oral solid dosage form containing berberine. The method overcomes manufacturing challenges mentioned in background prior art, i.e. undesirable physical properties of berberine substance such as poor flow properties and sticking problems, which cause severe difficulties during dosage form manufacturing, e.g. direct compression, wet granulation and even dry granulation processes, and therefore great loss of material during the manufacturing process. Surprisingly, it was found that berberine particles, when pre-mixed with a glidant or with a glidant with up to 20 wt % of a filler in the first homogenization step, show significantly improved physical properties such as powder flow and non-sticking behaviour. Using this procedure, berberine particles are covered by the glidant particles which are very small thus have a large surface area in total. A mixture obtained is then easily processed by the above mentioned conventional technology of direct tabletting or dry granulation.

The term "berberine" as used herein includes free berberine and pharmaceutically acceptable salts thereof.

The object of the present invention is a manufacturing method of oral dosage form containing berberine, which contains the following steps:
(i) homogenization of berberine with at least one glidant, the glidant optionally containing up to 20 wt% of a filler, to obtain a homogenous mixture A, wherein the particle size of at least 90 % of all berberine particles is preferably smaller than 500 µm, more preferably smaller than 50 µm, most preferably smaller than 10 µm with mean particle size of c. 5 µm; Preferably, the glidant specific surface area is in the range of from 100 to 400 m²/g, more preferably in the range of from 150 to 250 m²/g.
(ii) homogenization of the homogenous mixture A from step (i) with at least one filler to obtain a homogenous mixture B;
(iii) homogenization of the product from step (ii) (mixture B) with at least one lubricant to obtain a homogenous mixture C;
(iv) dry granulation and sieving of the homogenous mixture C from step (iii) to obtain a granulated mixture C;
(v) homogenization of the granulated mixture C from step (iv) with at least one disintegrant to obtain a homogenous mixture D;
(vi) preparation of oral dosage form selected from tablets, coated tablets, pills, orally disintegrating tablets, capsules, powders and/or granules using the homogenized mixture from step (v). Conventional methods are used for oral dosage form preparation in step (vii), and a person skilled in the art would know which method should be used.

Preferably, mixture D from step (v) is homogenized with at least one lubricant to obtain a homogenous mixture E before entering into step (vi).

The resulting homogenous mixture from step (vi) contains preferably from 100 to 1000 mg of berberine, from 2 to 100 mg of a glidant, from 50 to 1000 mg of a filler, from 5 to 70 mg of a lubricant and from 5 to 30 mg of a disintegrant. All ingredient amounts herein and hereafter are calculated per one dosage unit of the final dosage form (e.g. tablet or capsule). In a preferred embodiment, the resulting homogenous mixture from step (vi) contains 500 mg of berberine, 27 mg of a glidant, 332.5 mg of a filler, 22.5 mg of a lubricant and 9 mg of a disintegrant. In the most preferred embodiment, the resulting homogenous mixture from step (vi) contains 500 mg of berberine, 27 mg of colloidal anhydrous silica, 332.5 mg of microcrystalline silicified cellulose, 22.5 mg of magnesium stearate and 9 mg of sodium croscarmellose.

### Homogenization

Generally, homogenization processes are used in order to make a homogenous mixture of an active substance and excipients, which would be further suitable for following manufacturing steps such as granulation, compression, encapsulation and others. In the present invention, homogenization comprises several blending steps. The first step is pre-mixing of the active substance (berberine) with a glidant or pre-mixing of the active substance with a glidant and a filler or pre-mixing of the active substance with a glidant, a filler, a lubricant and a disintegrant. Various manufacturing equipment can be used for homogenization process, e.g. low speed blenders. A person skilled in the art would be familiar with conventional homogenization methods.

### Dry granulation and sieving

Dry granulation process is employed to form granules without using a liquid solution. Homogenized powder mixture is dry granulated using roller compactor or tablet press. Powder is squeezed between two rotating rollers to produce a continuous ribbon of materials in case of roller compaction. Powder is filled into the dies and compressed to tablets in case of tablet compression. Compressed material both ribbon or tablet is then milled to get granules with desired particle size. The desired particle size in step (iv) of the present invention corresponds to sieve sizes from 0.5 mm to 3.0 mm. used for milling. Final granules are used for following processing such as homogenization, compression, encapsulation, sachet filling and coating. A person skilled in the art would be familiar with conventional dry granulation and sieving methods.

In one prefered embodiment, the manufacturing method according to the present invention contains berberine and the glidant in step (i) in weight ratio from 500 : 1 to 5 : 1.

In one embodiment, the glidant is selected from colloidal anhydrous silica, talc and/or calcium silicate. Preferably, the glidant specific surface area is in the range of from 100 to 400 m²/g, more preferably in the range of from 150 to 250 m²/g.

In one embodiment, the filler is selected from microcrystalline silicified cellulose, microcrystalline cellulose, mannitol, sorbitol, lactose, calcium hydrogen phosphate, starch and pregelatinized starch.

In one preferred embodiment, the manufacturing method according to the present invention contains berberine and the filler in step (ii) in weight ratio from 20 : 1 to 1 : 10.

In one embodiment, the lubricant is selected from magnesium stearate, talc, calcium stearate, stearic acid, sodium stearyl fumarate, glycerol behenate, glycerol distearate.

In one preferred embodiment, the manufacturing method according to the present invention contains berberine and the lubricant are in weight ratio from 200: 1 to 5 : 1

In one embodiment of the manufacturing method according to the present invention, the dry granulation and sieving in step (iv) takes place using pressure in the range of from 10 bar to 150 bar (corresponding to the range of from 1 MPa to 15 MPa), roller gap in the range of from 0.5 to 5.0 mm and sieve size in the range of from 3.0 to 0.5 mm.

In one embodiment, the disintegrant is selected from sodium croscarmellose, crosslinked polyvinylpyrrolidone and sodium salt of carboxymethyl starch.

In one embodiment, oral dosage forms from the step (vii) are further coated with a coating material. Preferably, the content of the coating material in the oral dosage form is from 3 to 5 % (w/w), more preferably 4 % (w/w), of the total oral dosage form weight.

The coating materials are preferably selected from hydroxypropyl cellulose, hydroxypropyl methylcellulose, PEG (polyethylene glycol of molecular mass in the range of from 400 to 8000), TEG (triethylene glycol), propylene glycol, polyvinyl alcohol, polydextrose, titanium dioxide, talc, maltodextrin, triglycerides, colourants.

The manufacturing method of oral dosage form containing berberine may contain the following steps:
(i) homogenization of 100 to 1000 weight units of berberine with particle size smaller than 50 µm, preferably 500 weight units of berberine with particle size smaller than 50 µm, with 2 to 100 weight units of a glidant, preferably from 20 to 35 weight units of a glidant, most preferably the glidant is colloidal anhydrous silica, to obtain a homogenous mixture A;
(ii) homogenization of the homogenous mixture A from step (i) with 50 to 1000 weight units of a filler, preferably from 250 to 500 weight units of a filler, most preferably the filler is silicified microcrystalline cellulose, to obtain a homogenous mixture B;
(iii) homogenization of the product from step (ii) (mixture B) with 2 to 35 weight units of a lubricant, preferably from 5 to 15 weight units of a lubricant, most preferably the lubricant is magnesium stearate, to obtain a homogenous mixture C;
(iv) dry granulation and sieving of the homogenous mixture C to obtain a granulated mixture C;
(v) homogenization of the granulated mixture C from step (iv) with 5 to 30 weight units of a disintegrant, preferably from 5 to 15 weight units of a disintegrant, most preferably the disintegrant is sodium croscarmellose, to obtain a homogenous mixture D;
(vi) homogenization of the mixture D from step (v) with 2 to 35 weight units of a lubricant, preferably from 5 to 15 weight units of a lubricant, most preferably the lubricant is magnesium stearate, to obtain a homogenous mixture E;
(vii) preparation of oral dosage form selected from tablets, coated tablets, pills, orally disintegrating tablets, capsules, powders and/or granules using the homogenized mixture from step (vi). The preparation of the above mentioned oral dosage forms includes processing, such as dry granulation, homogenization, compression and capsule filling, obvious to the person skilled in the art. The resulting homogenous mixture from step (vi) contains from 100 to 1000 mg of berberine, from 2 to 100 mg of a glidant, from 50 to 1000 mg of a filler, from 5 to 70 mg of a lubricant and from 5 to 30 mg of a disintegrant. All ingredient amounts herein and hereafter are calculated per one dosage unit of the final dosage form (e.g. tablet or capsule).

The resulting oral dosage forms might be further coated with a coating material selected from hydroxypropyl cellulose, hydroxypropyl methylcellulose, PVA (polyvinylalcohol), PEG (polyethylene glycol of molecular mass in the range of from 400 to 8000), TEG (triethylene glycol), propylene glycol, polyvinyl alcohol, polydextrose, titanium dioxide, talc, maltodextrin, medium chain triglycerides, colourants.

Most preferably, the manufacturing method of oral dosage form containing berberine contains the following steps:
(i) homogenization of 500 weight units of berberine with particle size smaller than 50 µm with 27 weight units of colloidal anhydrous silica, to obtain a homogenous mixture A;
(ii) homogenization of the homogenous mixture A from step (i) with 332.5 weight units of silicified microcrystalline cellulose, to obtain a homogenous mixture B;
(iii) homogenization of mixture B from step (ii) with 11.25 weight units of magnesium stearate, to obtain a homogenous mixture C;
(iv) dry granulation and sieving of the homogenous mixture C to obtain a granulated mixture C;
(v) homogenization of the granulated mixture C from step (iv) with 9 weight units of sodium croscarmellose, to obtain a homogenous mixture D;
(vi) homogenization of the mixture D from step (v) with 11.25 weight units of magnesium stearate, to obtain a homogenous mixture E;
(vii) preparation of oral dosage form selected from tablets, coated tablets, pills, orally disintegrating tablets, capsules, powders and/or granules using the homogenized mixture from step (vi). The preparation of the above mentioned oral dosage forms includes processing, such as dry granulation, homogenization, compression and capsule filling, obvious to the person skilled in the art. The resulting homogenous mixture from step (vi) therefore contains 500 mg of berberine, 27 mg of colloidal anhydrous silica, 332.5 mg of silicified microcrystalline cellulose, 22.5 mg of magnesium stearate and 9 mg of sodium croscarmellose. All ingredient amounts herein and hereafter are calculated per one dosage unit of the final dosage form (e.g. tablet or capsule). The resulting oral dosage forms might be further coated with a coating material selected from hydroxypropyl cellulose, hydroxypropyl methylcellulose, PEG (polyethylene glycol of molecular mass in the range of from 400 to 8000), TEG (triethylene glycol), propylene glycol, polyvinyl alcohol, polydextrose, titanium dioxide, talc, maltodextrin, medium chain triglycerides, colourants.

The object of the present invention is also an oral dosage form containing berberine, which contains from 100 to 1000 mg of berberine (in a form of free berberine and/or a pharmaceutically acceptable salt thereof); from 2 to 100 mg of a glidant, selected from colloidal anhydrous silica, talc and/or calcium silicate; from 50 to 1000 mg of a filler, selected from microcrystalline silicified cellulose, microcrystalline cellulose, mannitol, lactose, starch and pregelatinized starch; from 5 to 70 mg of a lubricant, selected from magnesium stearate, talc, calcium stearate and stearic acid; and from 5 to 30 mg of a disintegrant, selected from sodium croscarmellose, polyvinylpyrrolidone and sodium salt of carboxymethyl starch. The oral dosage form is selected from tablets, coated tablets, pills, orally disintegrating tablets, capsules, powders and/or granules.

In one preferred embodiment, the oral dosage form of the present invention is obtainable by the manufacturing method of oral dosage form containing berberine of the present invention as described above.

In one preferred embodiment, the oral dosage form containing berberine contains 500 mg of berberine (in a form of free berberine and/or a pharmaceutically acceptable salt thereof); 27 mg of a glidant, selected from colloidal anhydrous silica, talc and/or calcium silicate; 332.5 mg of a filler, selected from microcrystalline silicified cellulose, microcrystalline cellulose, mannitol, lactose, starch and pregelatinized starch; 22.50 mg of a lubricant, selected from magnesium stearate, talc, calcium stearate and stearic acid; and 9 mg of a disintegrant, selected from sodium croscarmellose, polyvinylpyrrolidone and sodium salt of carboxymethyl starch. The oral dosage form is selected from tablets, coated tablets, pills, orally disintegrating tablets, capsules, powders and/or granules.

In the most prefered embodiment, the oral dosage form containing berberine, selected from tablets, coated tablets, pills, orally disintegrating tablets, capsules, powders and/or granules, contains 500 mg of berberine (in a form of free berberine and/or a pharmaceutically acceptable salt thereof); 27 mg of colloidal anhydrous silica; 332.5 mg of microcrystalline silicified cellulose; 22.50 mg of magnesium stearate; and 9 mg of sodium croscarmellose.

The object of the present invention is also the oral dosage form containing berberine according to the present invention for use as a medicament.

The object of the present invention is also the oral dosage form containing berberine according to the present invention for use in the treatment of metabolic syndrome, diabetes, prediabetes, dyslipidemia, hypertension, non-alcoholic fatty liver disease, obesity, gastrointestinal and other infections, diarrhoea including traveler s diarrhoea, irritable bowel syndrome, inflammatory bowel disease, polycystic ovary syndrome, heart diseases, cancers, neurodegenerative diseases.

The object of the present invention is also use of the oral dosage form containing berberine according to the present invention as a food supplement, preferably as a food supplement for blood glucose and lipids normalization, blood pressure normalization, weight reduction, normalization of intestinal functions including diarrhoea improvement, modulation of biochemical processes in the body.

### Examples

The invention is further illustrated by the following examples, which should not be construed as limiting the claimed scope.

Examples 1, 6, 8 and partially 4 and 5 present preferred embodiments of the invention. Examples 2, 3 and 9 do not present embodiments of the present invention, they illustrate inconvenient embodiments in order to compare the present invention with conventional methods used in the state of the art.

### Example 1: Preparation of oral dosage form of berberine using dry granulation - roller compaction

The contents of the resulting mixture are described in Table 1.

Example 1 presents an optimal composition and manufacturing process according to the present invention.

**Table 1: Composition of the oral dosage form of berberine according to the present invention**

| Composition | Theory 1 pc (mg) | % | Function |
|---|---|---|---|
| Berberine | 500.00 | 53.4 | Active substance |
| Cellulose, Silicified Microcrystalline (SMCC 90) | 332.5 | 35.5 | Filler |
| Silica, colloidal anhydrous (Aerosil) | 27.00 | 2.9 | Glidant |
| Croscarmellose sodium | 18.00 | 2.0 | Disintegrant |
| **Magnesium** stearate | 22.50 | 2.4 | Lubricant |
| **Core** | 900.0 | | |
| Opadry II 57220037 yellow | 36.0 | 3.8 | Coating |
| **Coated tablet** | 936.00 | 100.0 | |

### Manufacturing process:

The oral dosage form of berberine according to the present invention was prepared as follows:
Sieving: Magnesium stearate through sieve of 0.5 mm
   Silica, colloidal anhydrous through sieve of 1.0 mm
Homogenization of starting materials:
   1) Homogenization (pre-mixing) of berberine and silica, (homogenized mixture A). Low speed blender used at 15 RPM for 10 mins.
   2) Homogenization of A and silicified microcrystalline cellulose (homogenized mixture B). Low speed blender used at 15 RPM for 10 mins.
   3) Homogenization of B and ½ of total amount of magnesium stearate (homogenized mixture C). Low speed blender used at 15 RPM for 5 mins.
Dry granulation (roller compaction) and sieving of the homogenous mixture C from step (iii) to obtain a granulated mixture C.
   Compaction process parameters:
   Pressure 30 bar
   Roller gap 1.6 mm
   Sieves sizes 1.6 mm (1st sieve) + 0.8 mm (2nd sieve)
Homogenization of granules, disintegrant and lubricant
   4) Homogenization of granulated mixture C and croscarmellose sodium. Low speed blender used at 15 RPM for 10 mins. (resulting in a homogenized mixture D)
   5) Homogenization of homogenized mixture D and ½ of total amount of magnesium stearate. Low speed blender used at 15 RPM for 5 mins. (homogenized mixture E - final mixture ready for compression). Evaluated parameters of the final mixture are in Table 2.
Compression (Tabletting)
   Tablet core parameters: oblong 19 mm x 9 mm. Evaluated parameters of the tablet core are in Table 3.
   Tablet press: common rotary tabletting machine.
Coating of tablet cores
   Coating equipment: common coater for solid dosage forms such as tablets.
   Coating: 15% coating suspension

### Evaluation

Manufacturing process and composition of Example 1 presents the most advantageous and preferable example of the invention. All physical evaluated parameters mentioned below in Tables 2, 3 and 4 are very good.

**Table 2: Evaluated parameters of the final homogenized mixture E**

| | |
|---|---|
| Physical tests of final mixture E | batch 13-190117 |
| Bulk density | 0,5731 g/ml |
| Flow angle | 85,2° |
| Flowability | 11,87g/s |

**Table 3: Evaluated parameters of the final tablet core**

| | |
|---|---|
| Physical tests of tablet cores | batch 13-190117 |
| Weight | 901,3 mg |
| Friability | 0,14 % |
| Hardness | 208 N |
| Shape | 19x9 mm |

**Table 4: Evaluation of yield an losses during manufacturing**

| Evaluation of yield an losses during manufacturing | Real yield (% w/w of theoretical 100% yield) | Real losses (% w/w) |
|---|---|---|
| Yield of final mixture manufacturing* | 95 % | 5 % |
| Yield of tablet cores manufacturing* | 97 % | 3 % |

| | | |
|---|---|---|
| *Yield 95 % and more shows very low sticking or non-sticking problem during manufacture. | | |

### Example 2 (not part of the present invention) Preparation of oral dosage form of berberine using wet granulation - high shear

**Table 5: Composition of the oral dosage form of berberine according to batch nb. 04-070916**

| Composition | Theory 1 pc (mg) | % | Function |
|---|---|---|---|
| Berberine | 500.00 | 66.7 | Active substance |
| Croscarmellose sodium | 7.50 | 1.0 | Disintegrant |
| Cellulose, Silicified Microcrystalline (SMCC 90) | 197.50 | 26.3 | Filler |
| Magnesium stearate | 20.00 | 2.7 | Lubricant |
| Povidone 25 | 7.50 | 1.0 | Granulation aid |
| Glycerine | 7.50 | 1.0 | Granulation aid |
| Silica, colloidal anhydrous (Aerosil) | 10.00 | 1.3 | Glidant |
| **Weight of core:** | 750.00 | 100.0 | |

### Manufacturing process:

The oral dosage form of berberine according to batch nb. 04-070916 was prepared as follows:
Wet granulation:
1) mixing berberine and SMCC 90 (mixture A)
2) granulation of mixture A with povidone 25/glycerine/water solution

### Evaluation

Mixture containing berberine and excipients was stuck on surfaces of granulation vessel and following manufacturing steps such as drying, sieving, compression and coating were impossible to be done. There was 100% of real losses.

### Example 3 (not part of the present invention) Preparation of oral dosage form of berberine using wet granulation - high shear with higher amount of glidant

**Table 6: Composition of the oral dosage form of berberine according to batch nb. 06-200916**

| Composition | Theory 1 pc (mg) | % | Function |
|---|---|---|---|
| Berberine | 500.00 | 66.7 | Active substance |
| Croscarmellose sodium | 7.50 | 1.0 | Disintegrant |
| Cellulose, Silicified Microcrystalline (SMCC 90) | 192.50 | 25.6 | Filler |
| Magnesium stearate | 20.00 | 2.7 | Lubricant |
| Povidone 25 | 7.50 | 1.0 | Granulation aid |
| Glycerine | 7.50 | 1.0 | Granulation aid |
| Silica, colloidal anhydrous (Aerosil) | 15.00 | 2.0 | Glidant |
| **Weight of core:** | 750.00 | 100.0 | |

### Manufacturing process:

The oral dosage form of berberine according to batch nb. 06-200916 was prepared as follows:
Wet granulation
1) mixing berberine and SMCC 90 and silica (mixture A)
2) granulation of mixture A with povidone 25/glycerine/water solution

### Evaluation

Mixture containing berberine and excipients was stuck on surfaces of granulation vessel and following manufacturing steps such as drying, sieving, compression and coating were impossible to be done. There was 100% of real losses.

### Example 4 (not part of the present invention) Preparation of oral dosage form of berberine using of direct compressing of homogenous mixture

**Table 7: Composition of the oral dosage form of berberine according to batch nb. 07-200916**

| Composition | Theory 1 pc (mg) | % | Function |
|---|---|---|---|
| Berberine | 500.00 | 55.5 | Active substance |
| Cellulose, Silicified Microcrystalline | 341.50 | 38.0 | Filler |
| Silica, colloidal anhydrous (Aerosil) | 27.00 | 3.0 | Glidant |
| Croscarmellose sodium | 9.00 | 1.0 | Disintegrant |
| Magnesium stearate | 22.50 | 2.5 | Lubricant |
| **Weight of core:** | 900.00 | 100.0 | |

### Manufacturing process:

The oral dosage form of berberine according to batch nb. 07-200916 was prepared as follows:
Sieving: Silica, colloidal anhydrous through sieve of 1.0 mm
   magnesium stearate through sieve of 0.5 mm
   (all other ingredients if needed through sieve 1.0 mm)
Homogenization of starting materials for briquettes:
   1) Homogenization (pre-mixing) of berberine and silica, (homogenized mixture A). Low speed blender used at 15 RPM for 10 mins.
   2) Homogenization of A and silicified microcrystalline cellulose and croscarmellose sodium (homogenized mixture A with filler and disintegrant). Low speed blender used at 15 RPM for 10 mins.
   3) Homogenization of previous mixture and magnesium stearate (final mixture). Low speed blender used at 15 RPM for 5 mins.
Compression (Tabletting)
   Tablet core parameters: oblong 20 mm x 10 mm. Evaluated parameters of the tablet core are in Table 9.
   Tablet press: common rotary tabletting machine.

### Evaluation

Low content of the disintegrant is responsible for poor material flow and corresponding insufficient dosing of the mixture into dies of tabletting machine. The final tablet shape (oblong 20x10 mm) is quite big for patient compliance. On the other hand all other physical evaluated parameters mentioned below in Tables 8 and 9 are acceptable.

**Table 8: Evaluated parameters of the final homogenized mixture C**

| | |
|---|---|
| Physical tests of final mixture C | batch 07-200916 |
| Bulk density | 0.3822 g/ml |
| Flow angle | 66,7° |
| Flowability | 2.32g/s |

**Table 9 Evaluated parameters of the final tablet core**

| | |
|---|---|
| Physical tests of cores | batch 07-200916 |
| Weight | 903.7 mg |
| Friability | 0.0 % |
| Hardness | 163 N |
| Shape | 20x10 mm |

**Table 10: Evaluation of yield an losses during manufacturing**

| Evaluation of yield an losses during manufacturing | Real yield (% w/w of theoretical 100% yield) | Real losses (% w/w) |
|---|---|---|
| Yield of final mixture manufacturing* | 95 % | 5 % |
| Yield of tablet cores manufacturing* | 97 % | 3 % |

| | | |
|---|---|---|
| *Yield 95 % and more shows very low sticking or non-sticking problem during manufacture. | | |

### Example 5 (not part of the present invention) Preparation of oral dosage form of berberine by dry granulation using tablet press

**Table 11: Content of the oral dosage form of berberine according to batch nb. 11-071016**

| Composition | Theory 1 pc (mg) | % | Function |
|---|---|---|---|
| Berberine | 500.00 | 55.5 | Active substance |
| Cellulose, Silicified Microcrystalline (SMCC 90) | 341.50 | 38.0 | Filler |
| Silica, colloidal anhydrous (Aerosil) | 27.00 | 3.0 | Glidant |
| Croscarmellose sodium | 9.00 | 1.0 | Disintegrant |
| Magnesium stearate | 22.50 | 2.5 | Lubricant |
| **Weight of core:** | 900.00 | 100.0 | |

### Manufacturing process:

The oral dosage form of berberine according to batch nb. 11-071016 was prepared as follows:
Sieving: Silica, colloidal anhydrous through sieve of 1.0 mm
   magnesium stearate through sieve of 0.5 mm
   (all other ingredients if needed through sieve 1.0 mm)
Homogenization of starting materials:
   1) Homogenization (pre-mixing) of berberine and silica, (homogenized mixture A). Low speed blender used at 15 RPM for 10 mins.
   2) Homogenization of A and silicified microcrystalline cellulose and croscarmellose sodium (homogenized mixture for next step). Low speed blender used at 15 RPM for 10 mins.
   3) Homogenization of previously obtained mixture and ½ of total amount of magnesium stearate (homogenized mixture D). Low speed blender used at 15 RPM for 5 mins.
Dry granulation (by tablet press)
   Briquette/tablet parameters: round flat shape, diameter 12 mm, hardness 100N
   Briquette/tablet press: common rotary tabletting machine.
Milling of briquettes/tablets to obtain granulated mixture D
   Through grater sieve of 1.5 mm
Homogenization of sieved mixture D, disintegrant and lubricant
   4) Homogenization of screened mixture D and croscarmellose sodium. Low speed blender used at 15 RPM for 10 mins. (resulting in a homogenized mixture D with disintegrant)
   5) Homogenization of homogenized mixture D with disintegrant and ½ of total amount of magnesium stearate. Low speed blender used at 15 RPM for 5 mins. (homogenized mixture E - final mixture ready for compression). Evaluated parameters of the final mixture are in Table 12.
Compression (Tabletting) of mixture E
   Tablet core parameters: oblong 20x10 mm. Evaluated parameters of tablet cores are in table 13.
   Tablet press: common rotary tabletting machine.
Coating of tablet cores:
   Coating equipment: common coater for solid dosage forms such as tablets.
   Coating: 15% coating suspension

### Evaluation

Manufacturing process and composition of Example 5 present time-consuming process. Because of a poor flow the mixture before dry granulation needs to be filled into the tabletting machine manually. This final tablet shape (oblong 20x10 mm) is quite big for patient compliance. On the other hand all other physical evaluated parameters mentioned below in Tables 12and 13 are acceptable.

**Table 12: Evaluated parameters of the final homogenized mixture E (after dry granulation)**

| | |
|---|---|
| Physical tests of final mixture E | batch 11-071116 |
| Bulk density | 0.6116 g/ml |
| Flow angle | 85.0° |
| Flowability | 11.46 g/s |

**Table 13: Evaluated parameters of the final tablet cores**

| | |
|---|---|
| Physical tests of cores | batch 11-071116 |
| Weight | 899.5 mg |
| Friability | 0.13 % |
| Hardness | 131 N |
| Shape | 20x10 mm |

**Table 14: Evaluation of yield an losses during manufacturing**

| Evaluation of yield an losses during manufacturing | Real yield (% w/w of theoretical 100% yield) | Real losses (% w/w) |
|---|---|---|
| Yield of final mixture manufacturing* | 95 % | 5 % |
| Yield of tablet cores manufacturing* | 96 % | 4 % |

| | | |
|---|---|---|
| *Yield 95 % and more shows very low sticking or non-sticking problem during manufacture. | | |

### Example 6 Preparation of oral dosage form of berberine by dry granulation - roller compaction of mixture containing low amount of silica colloidal anhydrous

**Table 15: Composition of the oral dosage form of berberine according to batch nb. 15-160517**

| Composition | Theory 1 pc (mg) | % | Function |
|---|---|---|---|
| Berberine | 500.00 | 56.9 | Active substance |
| Cellulose, Silicified Microcrystalline (SMCC 90) | 332.50 | 37.9 | Filler |
| Silica, colloidal anhydrous (Aerosil) | 5.00 | 0.5 | Glidant |
| Croscarmellose sodium | 18.00 | 2.0 | Disintegrant |
| Magnesium stearate | 22.50 | 2.7 | Lubricant |
| **Weight of core:** | 878.00 | 100.0 | |

### Manufacturing process:

The oral dosage form of berberine according to batch nb. 15-160517 was prepared as follows:
Sieving: Silica, colloidal anhydrous through sieve of 1.0 mm
   magnesium stearate through sieve of 0.5 mm
   (all other ingredients if needed through sieve 1.0 mm)
Homogenization of starting materials:
   1) Homogenization (pre-mixing) of berberine and silica, (homogenized mixture A). Low speed blender used at 15 RPM for 10 mins.
   2) Homogenization of A and silicified microcrystalline cellulose (homogenized mixture B). Low speed blender used at 15 RPM for 10 mins.
   3) Homogenization of B and ½ of total amount of magnesium stearate (homogenized mixture C). Low speed blender used at 15 RPM for 5 mins.
Dry granulation (roller compaction) and sieving of the homogenous mixture C from step 3 to obtain a granulated mixture C.
   Compaction process parameters:
   Pressure 30 bar
   Roller gap 1.6 mm
   Sieves sizes 1.6 mm (1st sieve) + 0.8 mm (2nd sieve)
Homogenization of granules, disintegrant and lubricant
   4) Homogenization of granulated mixture C and croscarmellose sodium. Low speed blender used at 15 RPM for 10 mins. (resulting in a homogenized mixture D)
   5) Homogenization of homogenized mixture D and ½ of total amount of magnesium stearate. Low speed blender used at 15 RPM for 5 mins. (homogenized mixture E - final mixture ready for compression). Evaluated parameters of the final mixture are in Table 16.
Compression (Tabletting)
   Tablet core parameters: oblong 19 mm x 9 mm. Evaluated parameters of the tablet core are in Table 17.
   Tablet press: common rotary tabletting machine.

### Evaluation

Technological test of low amount of silica colloidal anhydrous in the mixture to show for patent variability. All physical evaluated parameters mentioned below in Tables 16 and 17 are acceptable.

**Table 16: Evaluated parameters of the final homogenized mixture E**

| | |
|---|---|
| Physical tests of final mixture E | batch 15-160517 |
| Bulk density | 0.5935 g/ml |
| Flow angle | 84.90° |
| Flowability | 11.13 g/s |

**Table 17: Evaluated parameters of the final tablet cores**

| | |
|---|---|
| Physical tests of cores | batch 15-160517 |
| Weight | 881.1 mg |
| Friability | 0.0 % |
| Hardness | 125 N |
| Shape | 19x9 mm |

**Table 18: Evaluation of yield an losses during manufacturing**

| Evaluation of yield an losses during manufacturing | Real yield (% w/w of theoretical 100% yield) | Real losses (% w/w) |
|---|---|---|
| Yield of final mixture manufacturing* | 95 % | 5 % |
| Yield of tablet cores manufacturing* | 96 % | 4 % |

| | | |
|---|---|---|
| *Yield 95 % and more shows very low sticking or non-sticking problem during manufacture. | | |

### Example 7 Preparation of oral dosage form of berberine by dry granulation - roller compaction - high hydraulic pressure during compaction

**Table 19: Composition of the oral dosage form of berberine according to batch nb. 16-160517/A**

| Composition | Theory 1 pc (mg) | % | Function |
|---|---|---|---|
| Berberine | 500.00 | 55.6 | Active substance |
| Cellulose, Silicified Microcrystalline (SMCC 90) | 332.50 | 36.9 | Filler |
| Silica, colloidal anhydrous (Aerosil) | 27.00 | 3.0 | Glidant |
| Croscarmellose sodium | 18.00 | 2.0 | Disintegrant |
| Magnesium stearate | 22.50 | 2.5 | Lubricant |
| **Weight of core:** | 900.00 | 100.0 | |

### Manufacturing process:

The oral dosage form of berberine according to batch nb. 16-160517/A was prepared as follows:
Sieving: Silica, colloidal anhydrous through sieve of 1.0 mm
   magnesium stearate through sieve of 0.5 mm
   (all other ingredients if needed through sieve 1.0 mm)
Homogenization of starting materials:
   1) Homogenization (pre-mixing) of berberine and silica, (homogenized mixture A). Low speed blender used at 15 RPM for 10 mins.
   2) Homogenization of A and silicified microcrystalline cellulose (homogenized mixture B). Low speed blender used at 15 RPM for 10 mins.
   3) Homogenization of B and ½ of total amount of magnesium stearate (homogenized mixture C). Low speed blender used at 15 RPM for 5 mins.
Dry granulation (roller compaction) and sieving of the homogenous mixture C from step 3) to obtain a granulated mixture C.
   Compaction process parameters:
   Pressure 100 bar
   Roller gap 1.6 mm
   Sieves sizes 1.6 mm (1st sieve) + 0.8 mm (2nd sieve)
Homogenization of granules, disintegrant and lubricant
   4) Homogenization of granulated mixture C and croscarmellose sodium. Low speed blender used at 15 RPM for 10 mins. (resulting in a homogenized mixture D)
   5) Homogenization of homogenized mixture D and ½ of total amount of magnesium stearate. Low speed blender used at 15 RPM for 5 mins. (homogenized mixture E - final mixture ready for compression). Evaluated parameters of the final mixture are in Table 20.
Compression (Tabletting)
   Tablet core parameters: oblong 19 mm x 9 mm. Evaluated parameters of the tablet core are in Table 21.
   Tablet press: common rotary tabletting machine.

### Evaluation

Due to a high hydraulic pressure during roller compaction, compressibility of material is exhausted and is insufficient for tablet core production. Tablet cores have very low hardness and high friability. All physical evaluated parameters are mentioned below in Tables 20 and 21.

**Table 20: Evaluated parameters of the final homogenized mixture E**

| | |
|---|---|
| Physical tests of final mixture E | batch 16-160517/A |
| Bulk density | 0.6849 g/ml |
| Flow angle | 86.60° |
| Flowability | 16.60 g/s |

**Table 21: Evaluated parameters of the final tablet cores**

| | |
|---|---|
| Physical tests of cores | batch 16-160517/A |
| Weight | 909.2 mg |
| Friability | 4.1 % |
| Hardness | 41 N |
| Shape | 19x9 mm |

**Table 22: Evaluation of yield an losses during manufacturing**

| Evaluation of yield an losses during manufacturing | Real yield (% w/w of theoretical 100% yield) | Real losses (% w/w) |
|---|---|---|
| Yield of final mixture manufacturing* | 95 % | 5 % |
| Yield of tablet cores manufacturing* | 0 % | 100 % |

| | | |
|---|---|---|
| *Yield 95 % and more shows very low sticking or non-sticking problem during manufacture. | | |

### Example 8 Preparation of oral dosage form of berberine by dry granulation - roller compaction - low amount of lubricant

**Table 23: Contents of the oral dosage form of berberine according to batch nb. 16-160517/B**

| Composition | Theory 1 pc (mg) | % | Function |
|---|---|---|---|
| Berberine | 500.00 | 56.3 | Active substance |
| Cellulose, Silicified Microcrystalline (SMCC 90) | 332.50 | 37.4 | Filler |
| Silica, colloidal anhydrous (Aerosil) | 27.00 | 3.0 | Glidant |
| Croscarmellose sodium | 18.00 | 2.0 | Disintegrant |
| Magnesium stearate | 11.25 | 1.3 | Lubricant |
| **Weight of core:** | 888.75 | 100.0 | |

### Manufacturing process:

The oral dosage form of berberine according to batch nb. 16-160517/B was prepared as follows:
Sieving: Silica, colloidal anhydrous through sieve of 1.0 mm
   magnesium stearate through sieve of 0.5 mm
   (all other ingredients if needed through sieve 1.0 mm
Homogenization of starting materials:
   1) Homogenization (pre-mixing) of berberine and silica, (homogenized mixture A). Low speed blender used at 15 RPM for 10 mins.
   2) Homogenization of A and silicified microcrystalline cellulose (homogenized mixture B). Low speed blender used at 15 RPM for 10 mins.
   3) Homogenization of B and ½ of total amount of magnesium stearate (homogenized mixture C). Low speed blender used at 15 RPM for 5 mins.
Dry granulation (roller compaction) and sieving of the homogenous mixture C from step 3 to obtain a granulated mixture C.
   Compaction process parameters:
   Pressure 30 bar
   Roller gap 1.6 mm
   Sieves sizes 1.6 mm (1st sieve) + 0.8 mm (2nd sieve)
Homogenization of granules, disintegrant and lubricant
   4) Homogenization of granulated mixture C and croscarmellose sodium. Low speed blender used at 15 RPM for 10 mins. (resulting in a final mixture D) Evaluated parameters of the final mixture are in Table 24.
Compression (Tabletting)
   Tablet core parameters: oblong 19 mm x 9 mm. Evaluated parameters of the tablet core are in Table 25.
   Tablet press: common rotary tabletting machine.

### Evaluation

Technological test of low amount of magnesium stearate for patent variability. All physical evaluated parameters are mentioned below in Tables 24 and 25.

**Table 24: Evaluated parameters of the final homogenized mixture D**

| | |
|---|---|
| Physical tests of final mixture D | batch 16-160517/B |
| Bulk density | 0.6116 g/ml |
| Flow angle | 84.90° |
| Flowability | 11.24 g/s |

**Table 25: Evaluated parameters of the final tablet cores**

| | |
|---|---|
| Physical tests of cores | batch 16-160517/B |
| Weight | 891.4 mg |
| Friability | 0.0 % |
| Hardness | 121 N |
| Shape | 19x9 mm |

**Table 26: Evaluation of yield an losses during manufacturing**

| Evaluation of yield an losses during manufacturing | Real yield (% w/w of theoretical 100% yield) | Real losses (% w/w) |
|---|---|---|
| Yield of final mixture manufacturing* | 95 % | 5 % |
| Yield of tablet cores manufacturing* | 95 % | 5 % |

| | | |
|---|---|---|
| *Yield 95 % and more shows very low sticking or complete removal of the sticking problem during manufacture. | | |

### Example 9 (comparative example) Preparation of oral dosage form of berberine by dry granulation - roller compaction - composition without glidant (silica)

**Table 27: Composition of the oral dosage form of berberine according to batch nb. 17-220517**

| Composition | Theory 1 pc (mg) | % | Function |
|---|---|---|---|
| Berberine | 500.00 | 55.6 | Active substance |
| Microcrystalline Cellulose (MCC 101) | 359.50 | 40.0 | Filler |
| Croscarmellose sodium | 18.00 | 2.0 | Disintegrant |
| Magnesium stearate | 22.50 | 2.5 | Lubricant |
| **Weight of core:** | 900.00 | 100.0 | |

### Manufacturing process:

The oral dosage form of berberine according to batch nb. 17-220517 was prepared as follows:
Sieving: magnesium stearate through sieve of 0.5 mm
   (all other ingredients if needed through sieve 1.0 mm)
Homogenization of starting materials:
   1) Homogenization (pre-mixing) of berberine and microcrystalline cellulose (homogenized mixture A). Low speed blender used at 15 RPM for 15 mins.
   2) Homogenization of A and ½ of total amount of magnesium stearate (homogenized mixture B). Low speed blender used at 15 RPM for 5 mins.
Dry granulation (roller compaction) and sieving of the homogenous mixture B from step 2 to obtain a granulated mixture C.
   Compaction process parameters:
   Pressure 30 bar
   Roller gap 1.6 mm
   Sieves sizes 1.6 mm (1st sieve) + 0.8 mm (2nd sieve)
Homogenization of granules, disintegrant and lubricant
   3) Homogenization of granulated mixture C and croscarmellose sodium. Low speed blender used at 15 RPM for 10 mins. (resulting in a homogenized mixture D)
   4) Homogenization of homogenized mixture D and ½ of total amount of magnesium stearate. Low speed blender used at 15 RPM for 5 mins. (homogenized mixture E - final mixture ready for compression). Evaluated parameters of the final mixture are in Table 28.
Compression (Tabletting)
   Tablet core parameters: oblong 19 mm x 9 mm. Evaluated parameters of the tablet core are in Table 29.
   Tablet press: common rotary tabletting machine.

### Evaluation

This technological test contains no glidant and MCC without silica as a filler. Homogenised mixture B has a very poor flow quality. Dry granulated mixture C could not be prepared without manually added mixture into the dies of tablet press. All physical evaluated parameters are mentioned below in Tables 28 and 29.

**Table 28: Evaluated parameters of the final homogenized mixture D**

| | |
|---|---|
| Physical tests of final mixture D | batch 17-220517 |
| Bulk density | 0.5747 g/ml |
| Flow angle | 84.70° |
| Flowability | 10.87 g/s |

**Table 29: Evaluated parameters of the final tablet cores**

| | |
|---|---|
| Physical tests of cores | batch 17-220517 |
| Weight | 904.2 mg |
| Friability | 0.0 % |
| Hardness | 144 N |
| Shape | 19x9 mm |

**Table 30: Evaluation of yield an losses during manufacturing**

| Evaluation of yield an losses during manufacturing | Real yield (% w/w of theoretical 100% yield) | Real losses (% w/w) |
|---|---|---|
| Yield of final mixture manufacturing* | 88 % | 12 % |
| Yield of tablet cores manufacturing* | 94 % | 6 % |

| | | |
|---|---|---|
| *Yield 95 % and more shows very low sticking or removal of the sticking problem during manufacture. | | |

Tablet cores can be prepared only by manually added mixture into the dies of tablet press. For commercial production this process is not feasible.

## Claims

1. Manufacturing method of oral dosage form containing berberine, **characterized in that** it contains the following steps:
(i) homogenization of berberine with at least one glidant, the glidant optionally containing up to 20 wt% of a filler, to obtain a homogenous mixture A;
(ii) homogenization of the homogenous mixture A from step (i) with at least one filler to obtain a homogenous mixture B;
(iii) homogenization of the homogenous mixture B from step (ii) with at least one lubricant to obtain a homogenous mixture C;
(iv) dry granulation and sieving of the homogenous mixture C from step (iii) to obtain a granulated mixture C;
(v) homogenization of the granulated mixture C with at least one disintegrant to obtain a homogenous mixture D;
(vi) preparation of oral dosage form selected from tablets, coated tablets, pills, orally disintegrating tablets, capsules, powders and/or granules using the homogenized mixture from step (v), whereas, optionally, berberine and the lubricant are in weight ratio from 200 : 1 to 5 : 1.

2. Manufacturing method according to claim 1, **characterized in that** berberine and the glidant in step (i) are in weight ratio from 500 : 1 to 5 : 1.

3. Manufacturing method according to any one of the preceding claims, **characterized in that** the glidant in step (i) is selected from colloidal anhydrous silica, talc and/or calcium silicate.

4. Manufacturing method according to any one of the preceding claims, **characterized in that** the filler is selected from microcrystalline silicified cellulose, microcrystalline cellulose, mannitol, sorbitol, lactose, calcium hydrogen phosphate, starch and pregelatinized starch.

5. Manufacturing method according to any one of the preceding claims, **characterized in that** berberine and the filler in step (ii) are in weight ratio from 20 : 1 to 1 : 10.

6. Manufacturing method according to any one of the preceding claims, **characterized in that** the lubricant is selected from magnesium stearate, talc, calcium stearate, stearic acid, sodium stearyl fumarate, glycerol behenate, glycerol distearate.

7. Manufacturing method according to any one of the preceding claims, **characterized in that** dry granulation and sieving in step (iv) takes place using pressure in the range of from 1 to 15 MPa, roller gap in the range of from 0.5 to 5.0 mm and sieve size in the range of from 3.0 to 0.5 mm.

8. Manufacturing method according to any one of the preceding claims, **characterized in that** the disintegrant is selected from sodium croscarmellose, crosslinked polyvinylpyrrolidon and sodium salt of carboxymethylstarch.

9. Manufacturing method according to any one of the preceding claims, **characterized in that** oral dosage forms from the step (vii) are further coated with a coating material.

10. Manufacturing method according to claim 9, **characterized in that** the coating material is selected from hydroxypropyl cellulose, hydroxypropyl methylcellulose, PEG, TEG, propylene glycol, polyvinylalcohol, polydextrose, titanium dioxide, talc, maltodextrin, triglycerides, colourants.

11. An oral dosage form containing berberine, selected from tablets, coated tablets, pills, orally disintegrating tablets, capsules, powders and/or granules, obtainable by the manufacturing method according to any one of the preceding claims 1 to 10, wherein berberine particles are covered by glidant particles, and the oral dosage form contains from 100 to 1000 mg of berberine; from 2 to 100 mg of a glidant, selected from colloidal anhydrous silica, talc and/or calcium silicate; from 50 to 1000 mg of a filler, selected from microcrystalline silicified cellulose, microcrystalline cellulose, mannitol, sorbitol, lactose, calcium hydrogen phosphate, starch and pregelatinized starch; from 5 to 70 mg of a lubricant, selected from magnesium stearate, talc, calcium stearate, stearic acid, sodium stearyl fumarate, glycerol behenate, glycerol distearate; and from 5 to 30 mg of a disintegrant, selected from sodium croscarmellose, crosslinked polyvinylpyrrolidon and sodium salt of carboxymethylstarch.

12. The oral dosage form containing berberine according to claim 11 for use as a medicament.

13. Use of the oral dosage form containing berberine according to claim 11 as a food supplement.

## Patentansprüche

1. Verfahren zur Herstellung einer Berberin enthaltenden oralen Darreichungsform, **dadurch gekennzeichnet, dass** es folgende Schritte enthält:
(i) Homogenisierung von Berberin mit mindestens einem Gleitmittel, wobei das Gleitmittel gegebenenfalls bis zu 20 Gew.-% eines Füllstoffs enthält, um eine homogene Mischung A zu erhalten;
(ii) Homogenisieren der homogenen Mischung A aus Schritt (i) mit mindestens einem Füllstoff, um eine homogene Mischung B zu erhalten;
(iii) Homogenisierung der homogenen Mischung B aus Schritt (ii) mit mindestens einem Schmiermittel, um eine homogene Mischung C zu erhalten;
(iv) Trockengranulierung und Siebung der homogenen Mischung C aus Schritt (iii), um eine granulierte Mischung C zu erhalten;
(v) Homogenisierung der granulierten Mischung C mit mindestens einem Sprengmittel, um eine homogene Mischung D zu erhalten;
(vi) Herstellung einer oralen Darreichungsform, ausgewählt aus Tabletten, Dragees, Pillen, oral zerfallenden Tabletten, Kapseln, Pulvern und/oder Granulaten unter Verwendung der homogenisierten Mischung aus Schritt (v), wobei gegebenenfalls Berberin und das Gleitmittel im Gewichtsverhältnis von 200 : 1 bis 5 : 1 sind.

2. Herstellungsverfahren nach Anspruch 1, **dadurch gekennzeichnet, dass** Berberin und das Gleitmittel in Schritt (i) in einem Gewichtsverhältnis von 500:1 bis 5:1 vorliegen.

3. Herstellungsverfahren nach einem der vorhergehenden Ansprüche, **dadurch gekennzeichnet, dass** das Gleitmittel in Schritt (i) aus kolloidaler wasserfreier Kieselsäure, Talkum und/oder Calciumsilikat ausgewählt wird.

4. Herstellungsverfahren nach einem der vorhergehenden Ansprüche, **dadurch gekennzeichnet, dass** der Füllstoff ausgewählt ist aus mikrokristalliner verkieselter Cellulose, mikrokristalliner Cellulose, Mannitol, Sorbitol, Lactose, Calciumhydrogenphosphat, Stärke und vorverkleisterter Stärke.

5. Herstellungsverfahren nach einem der vorhergehenden Ansprüche, **dadurch gekennzeichnet, dass** Berberin und der Füllstoff in Schritt (ii) in einem Gewichtsverhältnis von 20: 1 bis 1: 10 vorliegen.

6. Herstellungsverfahren nach einem der vorhergehenden Ansprüche, **dadurch gekennzeichnet, dass** das Gleitmittel ausgewählt ist aus Magnesiumstearat, Talkum, Calciumstearat, Stearinsäure, Natriumstearylfumarat, Glycerinbehenat, Glycerindistearat.

7. Herstellungsverfahren nach einem der vorhergehenden Ansprüche, **dadurch gekennzeichnet, dass** die Trockengranulierung und Siebung in Schritt (iv) unter Verwendung eines Drucks im Bereich von 1 bis 15 MPa, eines Walzenspalts im Bereich von 0,5 bis 5,0 mm und Siebgröße im Bereich von 3,0 bis 0,5 mm erfolgt.

8. Herstellungsverfahren nach einem der vorhergehenden Ansprüche, **dadurch gekennzeichnet, dass** das Sprengmittel ausgewählt ist aus Natriumcroscarmellose, vernetztem Polyvinylpyrrolidon und Natriumsalz der Carboxymethylstärke.

9. Herstellungsverfahren nach einem der vorhergehenden Ansprüche, **dadurch gekennzeichnet, dass** orale Darreichungsformen aus Schritt (vii) zusätzlich mit einem Beschichtungsmaterial beschichtet werden.

10. Herstellungsverfahren nach Anspruch 9, **dadurch gekennzeichnet, dass** das Beschichtungsmaterial ausgewählt ist aus Hydroxypropylcellulose, Hydroxypropylmethylcellulose, PEG, TEG, Propylenglykol, Polyvinylalkohol, Polydextrose, Titandioxid, Talkum, Maltodextrin, Triglyceride, Farbstoffe.

11. Berberinhaltige orale Darreichungsform, ausgewählt aus Tabletten, Dragees, Pillen, oral zerfallenden Tabletten, Kapseln, Pulvern und/oder Granulaten, erhältlich durch das Herstellungsverfahren nach einem der vorhergehenden Ansprüche 1 bis 10, wobei Berberinpartikel sind von gleitenden Partikeln bedeckt und die orale Darreichungsform enthält 100 bis 1000 mg Berberin; 2 bis 100 mg eines Gleitmittels, ausgewählt aus kolloidalem wasserfreiem Siliciumdioxid, Talkum und/oder Calciumsilicat; 50 bis 1000 mg eines Füllstoffs, ausgewählt aus mikrokristalliner verkieselter Cellulose, mikrokristalliner Cellulose, Mannitol, Sorbitol, Lactose, Calciumhydrogenphosphat, Stärke und vorverkleisterter Stärke; 5 bis 70 mg eines Gleitmittels, ausgewählt aus Magnesiumstearat, Talkum, Calciumstearat, Stearinsäure, Natriumstearylfumarat, Glycerinbehenat, Glycerindistearat; und 5 bis 30 mg eines Sprengmittels, ausgewählt aus Natriumcroscarmellose, vernetztem Polyvinylpyrrolidon und Natriumsalz von Carboxymethylstärke.

12. Berberinhaltige orale Darreichungsform nach Anspruch 11 zur Verwendung als Arzneimittel.

13. Verwendung der Berberinhaltigen oralen Darreichungsform nach Anspruch 11 als Nahrungsergänzungsmittel.

## Revendications

1. Un procédé de fabrication d'une forme galénique orale contenant de la berbérine, **caractérisé en ce qu'**il comporte les étapes suivantes :
(i) homogénéisation de la berbérine avec au moins un agent glissant, l'agent glissant contenant éventuellement jusqu'à 20 % en poids d'une charge, pour obtenir un mélange homogène A;
(ii) homogénéisation du mélange homogène A de l'étape (i) avec au moins une charge pour obtenir un mélange homogène B;
(iii) homogénéisation du mélange homogène B de l'étape (ii) avec au moins un lubrifiant pour obtenir un mélange homogène C;
(iv) granulation à sec et tamisage du mélange homogène C de l'étape (iii) pour obtenir un mélange granulé C;
(v) homogénéisation du mélange granulé C avec au moins un désintégrant pour obtenir un mélange homogène D;
(vi) préparation d'une forme galénique orale choisie parmi les comprimés, les comprimés enrobés, les pilules, les comprimés orodispersibles, les capsules, les poudres et/ou les granulés en utilisant le mélange homogénéisé de l'étape (v), tandis que, éventuellement, la berbérine et le lubrifiant sont en rapport de poids de 200 : 1 à 5 : 1.

2. Le procédé de fabrication selon la revendication 1, **caractérisé en ce que** la berbérine et l'agent glissant de l'étape (i) sont en rapport de poids de 500 : 1 à 5 : 1.

3. Le procédé de fabrication selon l'une quelconque des revendications précédentes, **caractérisé en ce que** l'agent glissant de l'étape (i) est choisi parmi la silice colloïdale anhydre, le talc et/ou le silicate de calcium.

4. Le procédé de fabrication selon l'une quelconque des revendications précédentes, **caractérisé en ce que** la charge est choisie parmi la cellulose microcristalline silicifiée, la cellulose microcristalline, le mannitol, le sorbitol, le lactose, l'hydrogénophosphate de calcium, l'amidon et l'amidon prégélatinisé.

5. Le procédé de fabrication selon l'une quelconque des revendications précédentes, **caractérisé en ce que** la berbérine et la charge de l'étape (ii) sont en rapport de poids de 20 : 1 à 1 : 10.

6. Le procédé de fabrication selon l'une quelconque des revendications précédentes, **caractérisé en ce que** le lubrifiant est choisi parmi le stéarate de magnésium, le talc, le stéarate de calcium, l'acide stéarique, le stéarylfumarate de sodium, le béhénate de glycérol, le distéarate de glycérol.

7. Le procédé de fabrication selon l'une quelconque des revendications précédentes, **caractérisé en ce que** la granulation à sec et le tamisage à l'étape (iv) ont lieu en utilisant une pression dans la plage de 1 à 15 MPa, un écart entre des rouleaux dans la plage de 0,5 à 5,0 mm et une taille de tamis dans la plage de 3,0 à 0,5 mm.

8. Le procédé de fabrication selon l'une quelconque des revendications précédentes, **caractérisé en ce que** le désintégrant est choisi parmi la croscarmellose de sodium, la polyvinylpyrrolidon réticulée et le sel de sodium du carboxyméthylamidon.

9. Le procédé de fabrication selon l'une quelconque des revendications précédentes, **caractérisé en ce que** les formes galéniques orales de l'étape (vii) sont en outre enrobées d'un matériau d'enrobage.

10. Le procédé de fabrication selon la revendication 9, **caractérisé en ce que** le matériau d'enrobage est choisi parmi l'hydroxypropylcellulose, l'hydroxypropylméthylcellulose, le PEG, le TEG, le propylène glycol, l'alcool polyvinylique, le polydextrose, le dioxyde de titane, le talc, la maltodextrine, les triglycérides, les colorants.

11. Une forme galénique orale contenant de la berbérine, choisie parmi les comprimés, les comprimés enrobés, les pilules, les comprimés orodispersibles, les capsules, les poudres et/ou les granulés, pouvant être obtenue par le procédé de fabrication selon l'une quelconque des revendications précédentes 1 à 10, dans laquelle les particules de berbérine sont recouverts de particules l'agent glissant, et la forme galénique orale contient de 100 à 1000 mg de berbérine; de 2 à 100 mg de l'agent glissant, choisi parmi la silice colloïdale anhydre, le talc et/ou le silicate de calcium; de 50 à 1 000 mg d'une charge, choisie parmi la cellulose microcristalline silicifiée, la cellulose microcristalline, le mannitol, le sorbitol, le lactose, l'hydrogénophosphate de calcium, l'amidon et l'amidon prégélatinisé; de 5 à 70 mg d'un lubrifiant, choisi parmi le stéarate de magnésium, le talc, le stéarate de calcium, l'acide stéarique, le stéarylfumarate de sodium, le béhénate de glycérol, le distéarate de glycérol; et de 5 à 30 mg d'un désintégrant, choisi parmi la croscarmellose de sodium, la polyvinylpyrrolidon réticulée et le sel de sodium du carboxyméthylamidon.

12. La forme galénique orale contenant de la berbérine selon la revendication 11 pour utilisation comme médicament.

13. Utilisation de la forme galénique orale contenant de la berbérine selon la revendication 11 comme supplément alimentaire.
